(19) Europäisches Patentamt
European Patent Office
Office européen des brevets

(11) EP 3 643 796 A1

(12) **EUROPEAN PATENT APPLICATION**

(43) Date of publication:
**29.04.2020 Bulletin 2020/18**

(51) Int Cl.:
***C12Q 1/689*** *(2018.01)*

(21) Application number: **18202627.8**

(22) Date of filing: **25.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **TBD-diagnostics GmbH**
**86853 Langerringen (DE)**

(72) Inventor: **Jenkins, Aikaterini**
**Hitchin SG5 2 PA (GB)**

(74) Representative: **SSM Sandmair Patentanwälte Rechtsanwalt Partnerschaft mbB**
**Joseph-Wild-Straße 20**
**81829 München (DE)**

(54) **DIAGNOSIS OF LYME DISEASE**

(57) Disclosed is a method and a composition for use in the detection, diagnosis, monitoring or prognostication of a *Borrelia* infection or for use in determining the progression of a *Borrelia* infection in an individual, wherein said method comprises the steps of recovering extracellular DNA, in particular exosomal DNA, of a bodily fluid sample of the individual to obtain a DNA sample; and determining the presence of at least one *Borrelia*-specific DNA biomarker in the DNA sample.

EP 3 643 796 A1

## Description

The present invention relates to the diagnosis of Lyme disease.

## Background

Lyme disease, also referred to as Lyme borreliosis, is an infectious disease caused by bacteria of the genus *Borrelia.* Lyme disease can be caused by different *Borrelia* species. In North America, *Borrelia burgdorferi* and *Borrelia mayonii* are the most prevalent causes of Lyme disease. In Europe and Asia, *Borrelia afzelii* and *Borrelia garinii* were also identified as causes of the disease.

Pathogenic *Borrelia* bacteria can be transmitted by ticks and upon infection an expanding area of reddened skin can appear at the site of the bite within a few days. This rash, known as erythema migrans, is typically neither itchy nor painful and although it is usually described as having a characteristic bulls eye appearance, it can often manifest in a different pattern and in varying sizes. In addition, approximately 25 to 50% of infected people do not develop such a rash, thus the patient can be unaware of the possibility of an infection. Other early indicators of a *Borrelia* infection may include unspecific symptoms such as fever, headache and feeling tired, often mistaken as symptoms of a cold or the flu.

If untreated, a *Borrelia* infection can cause joint pains, severe headaches along with neck stiffness, heart palpitations and loss of the ability to move one or both sides of the face, known as facial palsy. In untreated or persistent cases, repeated episodes of joint pain and swelling may occur months to years later and occasionally patients develop shooting pains or tingling in their arms and legs.

Lyme disease is the most common disease spread by ticks in the northern hemisphere. It is estimated that about 300,000 people in the United States and about 65,000 people in Europe are affected each year, with the real number of cases believed to be considerably higher, due to the lack of current diagnostic testing able to identify reliably all cases.

To prevent the development of Lyme disease, a course of doxycycline is usually prescribed when the patient presents with the characteristic bulls eye rash. However, this measure is not generally efficient since only a proportion of patients develop the rash even when they are infected and some might not recognize it as the characteristic type of rash. In addition, ticks can vary in size considerably, ranging from 1-2 millimeters in diameter (nymphs) to a few millimeters (adult ones) when un-engorged, with the nymphs more likely to pass on *Borrelia* bacteria. Thus, it is likely that patients might not associate a presenting rash with a tick bite as they can often be unnoticed.

It is generally believed that following a tick bite the development of an infection rarely occurs. If an infection is diagnosed early, a number of antibiotics can be effective, usually in combination, including doxycycline, amoxicillin, cefuroxime, rifampicin and azithromycin. Most people infected with *Borrelia* recover when treated with antibiotics taken orally within a few weeks of the infection. Despite treatment, about 10 to 20% of the patients go on to develop joint pains, loss of memory, and a general feeling of tiredness. This condition is often referred to as "Post-treatment Lyme Disease Syndrome" (PTLDS) and it can either be due to an ongoing infection, not fully resolved by the selected treatment protocol, thus resulting in a prolonged activation of the immune system post-treatment, or due to the development of an autoimmune disease. PTLDS is often included with untreated Lyme disease, under the more general term "Chronic Lyme Disease".

Being able to accurately diagnose an active *Borrelia* infection would be pivotal in determining a patient infected but without the typical rash or memory of a tick bite, as well as monitoring the effectiveness of the selected treatment protocol in each case.

For diagnostic purposes, *B. burgdorferi* can be grown in the laboratory, but this is generally not performed, as attempts to grow the pathogen from patient samples are rarely successful. Diagnosis of Lyme disease is currently based on a combination of symptoms, history of tick exposure, and testing for specific antibodies in the patient's blood. The commonly used serologic method for the diagnosis of Lyme disease is by detecting immunoglobulin M antibodies or an elevated titer of immunoglobulin G antibodies in an ELISA or with indirect immunofluorescence methods.

However, currently used blood tests are often negative in the early stages of the disease as it takes about 2 to 4 weeks post infection for antibodies against *Borrelia* to develop. Alternatively, anti-*Borrelia* antibodies may be diminished or never develop in the presence of a limited antibacterial treatment or due to the specific genetic make-up of the individual's immune system. This renders it impossible to correlate a detectable level of anti-*Borrelia* antibodies with any time frame of an infection with *Borrelia sp.* or active Lyme disease.

On the other hand, anti-*Borrelia* antibodies can be detected for many years after an infection occurred. Unfortunately, antibodies induced by an infection with *Borrelia sp.* are not protective against further exposures to *Borrelia* sp., therefore, a serologically positive result may not signify a current infection but indicate a prior infection. As such, a reinfection can easily be confused with the recurrence of an existing infection.

In addition, serological assays to detect *Borrelia sp.* may yield false-positive results because of cross-reactive antibodies that are associated with autoimmune disea-ses or with spirochetal, rickettsial, ehrlichial or other bacterial (e.g, *Helicobacter pylori*) infections.

As an alternative to serologic assays for the diagnosis of Lyme disease, poly-merase chain reaction (PCR) assays that detect *B. burgdorferi* DNA are available and are used more often in clinical settings. Their advantage over serological assays is the direct detection of infectious agents.

Current PCR assays can be used to detect *B. burgdorferi* DNA in blood, cerebrospinal fluid (CSF), urine or synovial fluid. The result of commonly used PCR assays is positive in approximately 30% of patients with active Lyme disease.

A notable disadvantage of current PCR testing is the likelihood of false-negative results because of the sparsity of large spirochete DNA fragments in samples and the use of more generic primers that may not be able to identify the specific *Borrelia sp.* of any given sample. Likewise, lack of extensive experience with the PCR technique can yield false-positive findings when insufficient care is taken to prevent environmental contamination.

Although most positive PCR results become negative within 2 weeks of adequate antimicrobial therapy, thus implying the efficient treatment of a patient, the results can remain positive for years after apparent cure. One of the most compelling uses of PCR can be in confirming persistent or recurrent disease, as a positive result is highly specific for confirming the presence of *B. burgdorferi.*

However, with the exception of synovial fluid, PCR testing is still not recommended for the diagnosis of Lyme disease because of low sensitivity, especially from the CSF, although it does have high specificity if the result is positive.

Therefore, there is a need for more specific and more sensitive PCR assays for the diagnosis of Lyme disease and/or detection of a *Borrelia* infection, in particular for PCR assays which further allow a correlation of the result with the disease status.

The object is solved by using *Borrelia* specific DNA Biomarkers that are present and prevalent as cell free DNA (extracellular DNA) in a bodily fluid sample of a subject. The invention is based on the surprising discovery that *Borrelia* specific DNA is present as extracellular and/or exosomal DNA in blood plasma or in exosomes of individuals affected by a *Borrelia* infection. The present invention utilizes *Borrelia* specific DNA that was found to be present as extracellular (i.e. cell free) DNA or exosomal DNA in blood plasma detecting, diagnosing, monitoring, prognosticating a *Borrelia* infection or the progression of a *Borrelia* infection.

## SUMMARY

In a first aspect, provided is a method for the detection, diagnosis, monitoring or prognostication of a *Borrelia* infection or the progression of a *Borrelia* infection in an individual.

In a second aspect, provided is a composition for use in the detection, diagnosis, monitoring or prognostication of a *Borrelia* infection or the progression of a *Borrelia* infection.

In a third aspect, provided is the use of nucleic acid molecule derived from a *Borrelia sp.* for detecting, diagnosing, monitoring or prognosticating a *Borrelia* infection or the progression of a *Borrelia* infection.

In a fourth aspect, provided is a method of identifying an individual for eligibility of a *Borrelia* infection therapy.

## DETAILED DESCRIPTION

According to the first aspect, provided is a method for the detection, diagnosis, monitoring or prognostication of a *Borrelia* infection or the monitoring or prognostication of the progression of a *Borrelia* infection in an individual. The method comprises the steps of recovering extracellular deoxyribonucleic acid molecules (DNA) from a bodily fluid sample obtained from the individual, and determining the presence and/or abundance of at least one *Borrelia-specific* DNA biomarker in the sample, and hence in the patient.

The method includes the step of recovering extracellular DNA from a bodily fluid sample of an individual. The bodily fluid sample may be selected from samples of the group consisting of urine, blood, blood plasma, saliva, tears sample, semen and spinal fluid.

The extracellular DNA of the bodily fluid sample may be recovered directly from the bodily fluid sample of the individual. In an additional and/or alternative embodiment, the extracellular DNA of the bodily fluid sample is recovered from exosomes that are present in the bodily fluid sample. More specifically, exosomes are recovered from the bodily fluid sample and subsequently the DNA that is present in the exosomes of the bodily fluid sample is recovered.

Exosomes are small vesicles that are released by various cells types. Exosomes have a diameter of about 40 to 100 nm and are generated intracellularly when a segment of the cell membrane invaginates and is endocytosed. The internalized segment of the cell membrane is broken into smaller vesicles that are subsequently expelled from the cell. Depending on their cellular origin, exosomes possess characteristic profiles of proteins and/or nucleic acids which can trigger signaling pathways in other cells or transfer exosomal constituents to other cells.

For recovering the extracellular DNA from exosomes, the exosomes within the bodily fluid sample are recovered from said bodily fluid sample to obtain a preparation of exosomes before the DNA is recovered from the preparation of exosomes. The DNA being recovered from exosomes is extracellular DNA.

The method further comprises the step of determining the presence and/or abundance of at least one *Borrelia-specific* DNA biomarker in the DNA sample.

The term "*Borrelia*-specific DNA biomarker" as used herein refers to DNA that originates from a *Borrelia* genome. Preferably, said "*Borrelia*-specific DNA biomarker" is a DNA that comprises a nucleotide sequence that is unique for species of the genus *Borrelia* or even unique for members of a specific *Borrelia* species. The term "*Borrelia*-specific DNA biomarker" as used herein refers to a DNA molecule that originates from the genetic material of bacteria from the genus *Borrelia.* Said "*Borrelia*-specific DNA biomarker" is a nucleotide sequence within the DNA molecule that is unique for species of the genus *Borrelia* or even unique for members of a specific *Borrelia* species. Thus, said nucleotide sequence differs from the nucleotide sequence of corresponding DNA molecules that originate from the individual, i.e. a mammal, preferably a human. Preferably, the nucleotide sequence of the *Borrelia-specific* DNA biomarker also differs from corresponding DNA molecules originating from other spirochetal, rickettsial, ehrlichial or other bacterial species. The term "corresponding DNA molecules" is meant to designate DNA molecules comprising nucleotide sequences which are homologs (i.e. descending from a common ancestral nucleotide sequences) as well as analogs (i.e. descending from different ancestral nucleotide sequences but having the same function) and orthologs (i.e. having evolved from the same common ancestor nucleotide sequence).

In an additional and/or alternative embodiment, the *Borrelia-specific* DNA biomarker is a DNA molecule comprising or consisting of a nucleotide sequence consisting of at least 10 consecutive nucleotides of one of the nucleotide sequences set forth in one of nucleotide sequences as represented by SEQ ID NO: 1 to SEQ ID NO: 5.

The presence and/or abundance of at least one *Borrelia-specific* DNA biomarker can be assessed by a variety of suitable methods.

For example, determining the presence and/or abundance of at least one *Borrelia*-specific DNA biomarker may be assessed by separation of the DNA molecules obtained from the bodily fluid sample in agarose or polyacrylamide gels, followed by contacting the thus separated DNA molecules with a DNA affinity ligand that is specific for at least one of the *Borrelia-specific* DNA biomarkers. In an additional andor alternative embodiment, said DNA affinity ligand is an oligonucleotide that is capable of hybridizing to the *Borrelia-specific* DNA biomarker. Hence, assessing the presence and/or abundance of a *Borrelia*-specific DNA biomarker comprises hybridization of the DNA molecules obtained from the bodily fluid sample with one or more oligonucleotide probes that are specific for the *Borrelia-specific* DNA biomarker.

Alternatively, the presence and/or abundance of the at least one *Borrelia-specific* DNA biomarker may be determined by labelling the DNA obtained from the bodily fluid sample followed by separation on a sequencing gel. DNA samples may be placed on the gel such that patient and control or standard DNA are in adjacent lanes. Assessment may be accomplished visually or by means of a densitometer.

Alternatively, the presence and/or abundance of the at least one *Borrelia-specific* DNA biomarker may be detected in a DNA array or microarray approach. Typically, sample DNA derived from subjects to be tested are processed and labeled, preferably with a fluorescent label. Subsequently, such nucleic acid molecules may be used in a hybridization approach with immobilized capture probes corresponding to the *Borrelia-specific* DNA biomarker of the present invention. Suitable means for carrying out microarray analyses are known to the person skilled in the art.

In a standard setup, a DNA array or microarray comprises immobilized high-density probes to detect a number of genes. The probes on the array are complementary to one or more parts of the sequence of the marker DNA. In the present invention, any type of *Borrelia-specific* DNA biomarker associated polynucleotide may be used as probe for the DNA array, as long as the polynucleotide allows for a specific distinction between the *Borrelia-specific* DNA biomarker and other genes. Typically, cDNAs, PCR products, and oligonucleotides are useful as probes. Preferably, a probe involving the specific portions of the *Borrelia*-specific DNA biomarker may be used as a probe. In addition to the determination of the presence and/or abundance of the *Borrelia*-specific DNA biomarker the determination of the presence and/or abundance of other genes, e.g. additional biomarker may be accomplished.

A DNA array- or microarray-based detection method typically comprises the following steps: (1) Isolating DNA from a bodily fluid sample, optionally amplifying at least a portion of the *Borrelia*-specific DNA-marker and subsequently labeling this DNA. Methods for labeling nucleic acids are described in manuals for micro array technology. (2) Hybridizing the DNA from step 1 with probes for the marker DNA. The DNA from a sample can be labeled with a dye, such as the fluorescent dyes Cy3 (red) or Cy5 (blue). Generally, a control sample is labeled with a different dye. (3) Detecting the hybridization of the nucleic acids from the sample with the probes and determining at least qualitatively, if not also quantitatively, the amounts of one or more of the *Borrelia*-specific DNA biomarkers and/or additional marker genes investigated. The difference in the abundance level between sample and control can be estimated based on a difference in the signal intensity. These can be measured and analyzed by appropriate software such as, but not limited to the software provided for example by Affymetrix.

There is no limitation on the number of probes corresponding to the *Borrelia*-specific DNA biomarkers used, which are spotted on a DNA array. Also, a *Borrelia*-specific DNA biomarker can be represented by two or more probes, the probes hybridizing to different parts of the *Borrelia*-specific DNA biomarker. Probes are designed for each selected *Borrelia*-specific DNA biomarker. Such a probe is typically an oligonucleotide comprising 5-50 nucleotide residues.

Longer DNAs can be synthesized by PCR or chemically. Methods for synthesizing such oligonucleotides and applying them on a substrate are well known in the field of micro-arrays. DNA other than the *Borrelia*-specific DNA biomarker(s) may be also spotted on the DNA array to normalize assay results or to compare assay results of multiple arrays or different assays.

Alternatively, the presence and/or abundance of one or more of the *Borrelia*-specific DNA biomarkers may be detected in a quantitative PCR (qPCR) approach, in a real-time qPCR approach. Preferably, Taqman or Molecular Beacon probes as principal FRET-based probes of this type may be used for quantitative PCR detection. In both cases, the *Borrelia*-specific DNA probes as defined herein, serve as internal probes which are used in conjunction with a pair of opposing primers that flank the target region of interest, preferably a set of *Borrelia*-specific DNA biomarker oligonucleotides as defined herein. Upon amplification of a target segment, the probe may selectively bind to the products at an identifying sequence in between the primer sites, thereby causing increases in FRET signaling relative to increases in target frequency.

Preferably, a Taqman probe to be used for a quantitative PCR approach according to the present invention may comprise a *Borrelia*-specific DNA biomarker oligonucleotide as defined herein of about 22 to 30 bases that is labeled on both ends with a FRET pair. Typically, the 5' end will have a shorter wavelength fluorophore such as fluorescein (e.g. FAM) and the 3' end is commonly labeled with a longer wavelength fluorescent quencher (e.g. TAMRA) or a non-fluorescent quencher compound (e.g. Black Hole Quencher). It is preferred that the *Borrelia*-specific DNA biomarker probes as defined herein, have no guanine (G) at the 5' end adjacent to the reporter dye in order to avoid quenching of the reporter fluorescence after the probe is degraded.

A Molecular Beacon probe to be used for a quantitative PCR approach according to the present invention preferably uses FRET interactions to detect and quantify a PCR product, with each probe having a 5' fluorescent-labeled end and a 3' quencher-labeled end. This hairpin or stem-loop configuration of the probe structure comprises preferably a stem with two short self-binding ends and a loop with a long internal target-specific region of about 20 to 30 bases.

Alternative detection mechanisms which may also be employed in the context of the present invention are directed to a probe fabricated with only a loop structure and without a short complementary stem region. An alternative FRET-based approach for quantitative PCR which may also be used in the context of the present invention is based on the use of two hybridization probes that bind to adjacent sites on the target wherein the first probe has a fluorescent donor label at the 3' end and the second probe has a fluorescent acceptor label at its 5' end.

In an additional and/or alternative embodiment, the *Borrelia*-specific DNA biomarker is a DNA molecule comprising a nucleotide sequence consisting of at least 10, at least 15, at least 20, at least 25, at least 30, at least 40, at least 50, at least 75, at least 100, at least 200, at least 300, at least 400, at least 500 consecutive nucleotides of one of the nucleotide sequences set forth in one of the nucleotides sequences selected from the group consisting of SEQ ID NO. 1 to SEQ ID NO. 5 (i.e. SEQ ID NO. 1, SEQ ID NO. 2, SEQ ID NO. 3, SEQ ID NO. 4 and SEQ ID NO. 5). The nucleotide sequences set forth in any one of SEQ ID NO. 1 to SEQ ID NO. 5 are *Borrelia* specific nucleotide sequences, and were found to be present as extracellular DNA in bodily fluid samples of individuals infected with *Borrelia sp.*

The nucleotide sequence according to SEQ ID NO. 1 represents the or at least a portion of the *B. burgdorferi che*R gene. The *B. burgdorferi che*R gene encodes a chemotaxis protein methyltransferase (CheR).

The nucleotide sequence as represented by SEQ ID NO. 2 represents the or at least a portion of the *B. burgdorferi pkf*B gene.

The nucleotide sequence as represented by SEQ ID NO. 3 represents the or at least a portion of the *B. burgdorferi uvrB* gene. The *B. burgdorferi* uvrB gene encodes a nucleotide excision repair protein (UvrB).

The nucleotide sequence as represented by SEQ ID NO. 4 represents the or at least a portion of the putative *B. burgdorferi* BB173 gene. The *B. burgdorferi* BB173 gene encodes an inner membrane protein of yet unknown function.

The nucleotide sequence as represented by SEQ ID NO. 5 represents the or at least a portion of the putative *B. burgdorferi* BB0037 gene. The *B. burgdorferi* BB173 gene encodes a protein of unknown function.

Nucleic acid molecules representing fragments of the nucleic acid molecules as represented by any one of SEQ ID No. 1 to SEQ ID NO. 5 were revealed as prevalent *Borrelia*-specific DNA biomarkers in extracellular and/or exosomal DNA retrieved from blood plasma of individuals diagnosed with Lyme disease.

In an additional and/or alternative embodiment, the presence and/or abundance of the *Borrelia*-specific DNA biomarker in the bodily fluid sample is determined and based on the presence and/or abundance of the *Borrelia*-specific DNA biomarker in the bodily fluid sample it is decided whether *Borrelia* is present in the individual and/or on the prevalence of *Borrelia* in the sample and/or the individual.

The presence and/or abundance of the *Borrelia*-specific DNA biomarker in the bodily fluid sample is determined in that the DNA of the DNA sample is contacted with at least one nucleic acid affinity ligand which is specific for the *Borrelia*-specific DNA biomarker. The term "specific" as used herein with respect to the nucleic acid affinity ligand refers to nucleic acid affinity ligands which do bind to DNA that originates from a *Borrelia* genome, but does not bind to DNA that originates from the individual's genome. Preferably, the affinity ligand being specific for the *Borrelia*-specific DNA biomarker does not bind to DNA that originates from any other microbial species than *Borrelia.*

In an additional and/or alternative embodiment, at least one nucleic acid affinity ligand is an oligonucleotide.

The term "oligonucleotide" as used herein refers to synthetic nucleic acid molecules having a length of at least 10, at least 15, at least 20 nucleotides, but less than 100, preferably less than 75, more preferably less than 50 nucleotides.

In an additional and/or alternative embodiment, the at least one oligonucleotide selected from the group consisting of oligonucleotides which

a) comprise a nucleotide sequence as represented in any one of SEQ ID NO: 1 to SEQ ID NO: 5;
b) comprise a nucleotide sequence having a sequence similarity of at least 80% to any one of the nucleotide sequences according to a);
c) comprise a nucleotide sequence being complementary to any one of the nucleotide sequences according to a) or b); and
d) which hybridize to any one the nucleic acid molecules comprising a nucleotide sequence of at least 15 consecutive nucleotides as present in the nucleotide sequences set forth in any one of SEQ ID NO: 1 to SEQ ID NO: 5 under stringent conditions.

It is to be understood that the nucleic acid affinity ligand or oligonucleotide being specific for the *Borrelia*-specific DNA biomarker can be used as a probe in the methods for determining the presence and/or abundance of the *Borrelia*-specific DNA biomarker.

In a further preferred embodiment, said method comprises the additional step of comparing the measured nucleic acid levels to a control level. The term "control level" (or "control state"), as used herein, relates to an amount of DNA in the bodily fluid sample which may have been determined at an earlier point of time and/or under similar or comparable conditions as the test sample by using previously collected and stored from the/an individual whose disease state is/are known. The term "disease state" relates to any state of *Borrelia* infection. Preferably the term includes different progression stages of a *Borrelia* infection.

Alternatively, the control level may be determined by a statistical method based on the results obtained by analyzing previously determined abundance level(s) of the *Borrelia*-specific DNA biomarker from previously tested individuals whose disease state is known. Furthermore, the control level can be derived from a database of abundance levels for *Borrelia*-specific biomarkers from previously tested individuals.

In an additional and/or alternative embodiment, the method comprises the use of two oligonucleotides.

In an additional and/or alternative embodiment, the DNA affinity ligand is modified in that the DNA affinity ligand is labeled to become readily detectable. The DNA affinity ligand may be modified with a fluorescent label and/or a radio label. Suitable labels are described herein below.

According to the second aspect, provided is a composition for use in the diagnosis, detection, monitoring or prognostication of a *Borrelia* infection, or for use in monitoring or prognosticating the progression of a *Borrelia* infection, wherein the composition contains at least one *Borrelia*-specific nucleic acid affinity ligand.

In an additional and/or alternative embodiment, the at least one *Borrelia*-specific nucleic acid affinity ligand is selected from the group consisting of at least one oligonucleotide selected from the group consisting of oligonucleotides which

a) comprise a nucleotide sequence as represented in any one of SEQ ID NO: 1 to SEQ ID NO: 5;
b) comprise a nucleotide sequence having a sequence similarity of at least 80% to any one of the nucleotide sequences according to a);
c) comprise a nucleotide sequence being complementary to any one of the nucleotide sequences according to a) or b); and
d) which hybridize to any one the nucleic acid molecules comprising a nucleotide sequence of at least 15 consecutive nucleotides as present in the nucleotide sequences set forth in any one of SEQ ID NO: 1 to SEQ ID NO: 5 under stringent conditions.

In an additional and/or alternative embodiment, the *Borrelia*-specific DNA affinity ligand may be labeled with various markers or may be detected by a secondary affinity ligand, labeled with various markers, to allow detection, visualization and/or quantification. This can be accomplished using any suitable labels, which can be conjugated to the affinity ligand capable of interaction with the *Borrelia*-specific DNA biomarker, using any suitable technique or methods known to the person skilled in the art. The term "secondary affinity ligand" refers to a molecule which is capable of binding to the affinity ligand as defined herein above (i.e. a "primary affinity ligand" if used in the context of a system with two interacting affinity ligands). The binding interaction is preferably a specific binding.

Examples of labels that can be conjugated to a primary and/or secondary affinity ligand include fluorescent dyes or metals (e.g. fluorescein, rhodamine, phycoerythrin, fluorescamine), chromophoric dyes (e.g. rhodopsin), chemiluminescent compounds (e.g. luminal, imidazole), bioluminescent proteins (e.g. luciferin, luciferase), and haptens (e.g.

biotin).

In a particularly preferred embodiment, an affinity ligand to be used as a probe may be labeled with a fluorescent label like 6-FAM, HEX, TET, ROX, Cy3, Cy5, Texas Red or Rhodamine, and/or at the same time with a quenching label like TAMRA, Dabcyl, Black Hole Quencher, BHQ-1 or BHQ-2. A variety of other useful fluorescents and chromophores are described in Stryer, 1968, Science, 162:526-533. Affinity ligands may also be labeled with enzymes (e.g. horseradish peroxidase, alkaline phosphatase, beta-lactamase), radioisotopes (e.g. $^{3}H$, $^{14}C$, $^{32}P$, $^{33}P$, $^{35}S$, $^{125}I$, $^{11}C$, $^{13}N$, $^{15}O$, $^{18}F$, $^{64}Cu$, $^{62}Cu$, $^{124}I$, $^{76}Br$, $^{82}Rb$, $^{68}Ga$ or $^{18}F$) or particles (e.g. gold).

The different types of labels may be conjugated to an affinity ligand using various chemistries, e.g. the amine reaction or the thiol reaction. However, other reactive groups than amines and thiols can also be used, e.g. aldehydes, carboxylic acids and glutamine.

In a specific embodiment of the present invention a composition may additionally comprise accessory ingredients like PCR buffers, dNTPs, a polymerase, ions like bivalent cations or monovalent cations, hybridization solutions, secondary affinity ligands like, e.g. secondary antibodies, detection dyes and any other suitable compound or liquid necessary for the performance of a detection based on any of the affinity ligands or contrast agents as defined herein above, which is known to the person skilled in the art.

In a preferred embodiment of the present invention a composition as defined herein above is a diagnostic composition.

In another aspect the present invention relates to a diagnostic kit for detecting, diagnosing, monitoring or prognosticating a *Borrelia* infection or the progression of a Borrelia infection, comprising a set of oligonucleotides specific for at least one of the *Borrelia*-specific DNA biomarker, a probe specific for the *Borrelia*-specific DNA biomarker.

Typically, the diagnostic kit of the present invention contains one or more agents allowing the specific detection of a *Borrelia*-specific DNA biomarker as defined herein. The agents or ingredients of a diagnostic kit may, according to the present invention, be comprised in one or more containers or separate entities. The nature of the agents is determined by the method of detection for which the kit is intended. The agents to be comprised may be a set of oligonucleotides specific for the *Borrelia* specific DNA biomarker as defined herein above, which may be optionally labeled according to methods known in the art, e.g. with labels described herein.

Typically, a diagnostic kit for the detection of *Borrelia*-specific DNA biomarker may comprise accessory ingredients like a PCR buffers, dNTPs, a polymerase, ions like bivalent cations or monovalent cations, hybridization solutions etc. A diagnostic kit for the detection of *Borrelia*-specific DNA biomarker may also comprise accessory ingredients like secondary affinity ligands, e.g. secondary antibodies, detection dyes and any other suitable compound or liquid necessary for the performance of a protein detection based known to the person skilled in the art. Such ingredients are known to the person skilled in the art and may vary depending on the detection method carried out. Additionally, the kit may comprise an instruction leaflet and/or may provide information as to the relevance of the obtained results.

According to the third aspect, provided is the use of a DNA molecule comprising a stretch of at least 10, at least 15, at least 20 consecutive nucleotides present in at least one nucleotide sequence set forth in SEQ ID NO. 1 to 5 as a *Borrelia*-specific biomarker for diagnosing, detecting, monitoring or prognosticating a *Borrelia* infection. The nucleotide sequences set forth in any one of SEQ ID NOs. 1 to 5 represent *Borrelia*-specific DNA biomarkers that are prevalent among the extracellular and/or exosomal DNA of plasma samples from patients infected with *Borrelia* sp. Hence, the nucleotide sequences set forth in any one of SEQ ID NOs. 1 to 5 are suitable DNA biomarkers indicating an infection with *Borrelia* sp. The DNA molecule comprising a stretch of at least 10 consecutive nucleotides being present in at least one nucleotide sequence set forth in SEQ ID NO. 1 to 5 may therefore be used as a DNA affinity ligand for the specific detection of a DNA molecule as present by the nucleotide sequences set forth in any one of SEQ ID NO: 1 to SEQ ID NO: 5 or fragments thereof provided that the DNA affinity ligand can specifically hybridize to the DNA molecule represented by one of SEQ ID NO: 1 to SEQ ID NO: 5, or fragments thereof.

In a preferred embodiment the present invention relates to the use of a set of oligonucleotides specific for the *Borrelia*-specific DNA biomarker, as defined herein above, for the preparation of a composition for diagnosing, detecting, monitoring or prognosticating a *Borrelia* infection or the progression of a *Borrelia* infection in an individual, as described herein above.

In an additional and/or alternative embodiment, provided is a method for diagnosing, detecting, monitoring or prognosticating a *Borrelia* infection, the method comprising at least the step of determining the presence and/or level of a *Borrelia*-specific DNA biomarker in a DNA sample containing extracellular DNA of a bodily fluid sample of a subject.

In an additional and/or alternative embodiment, the *Borrelia*-specific DNA biomarker is selected from the group consisting of one or more of the genes encoding CheR, PkfB, UvrB, BB0173 and/or BB0037, or fragments thereof.

In an additional and/or alternative embodiment, the method comprises the step of comparing the DNA level determined in the bodily fluid sample to a control level. The control level may be the DNA level measured in a bodily fluid sample of the same individual which was obtained from the individual at an earlier point of time. Alterations in the DNA level indicate the progression of a *Borrelia* infection.

According to the fourth aspect, provided is a method of identifying an individual for eligibility for a *Borrelia* infection therapy comprising:

(a) testing in a bodily fluid sample obtained from an individual for the presence and/or abundance of a *Borrelia*-specific DNA biomarker among the extracellular DNA present in said bodily fluid sample;
(b) classifying the levels of abundance of step (a); and
(c) identifying the individual as eligible to receive a *Borrelia* infection therapy where the individual's sample is classified as containing a *Borrelia*-specific DNA biomarker.

The present invention will be described with respect to particular embodiments, but the invention is not limited thereto but only by the claims.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be understood that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but they may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

**Example 1-Isolation of DNA**

Serum samples from 6 different individuals affected with Lyme disease were used to isolate circulating cell-free (i.e. extracellular) DNA and exosomal DNA.

The cell-free DNA was isolated from the samples using the QIAseq cfDNA all in one kit (QIAGEN GmbH, Hilden, DE) according to the manufacturer's recommendations. Cell free DNA was finally eluted in 60 μl AVE buffer.

The exosomal DNA was isolated from the samples using the exoEasy Maxi Kit (QIAGEN GmbH, Hilden, DE) according to the manufacturer's recommendations to isolate extracellular vesicles. Exosomes were eluted in 1 ml of buffer XE.

Subsequently, the DNA was isolated from the exosomes using the DNEasy Blood and Tissue kit (QIAGEN GmbH, Hilden, DE) including a proteinase K digest and a RNA removal step. The protocol differed from the manufacturers recommendations in that sequential centrifugations of the final lysate through the DNeasy mini spin columns were performed as the lysates' volumes were 1 ml while the maximum capacity of the DNeasy mini spin columns is restricted to 700 μl.

An aliquot of each DNA sample was used to determine the DNA concentration. The Qubit dsDNA HS Assay kit (Invitrogen) was used to determine the concentration in 1 μl of each aliquot in comparison to a given standard provided with the kit. The DNA concentration was calculated by applying the mathematical equation of linear regression on a linear trend line that was created.

Prior to the preparation of DNA libraries, exosomal DNA was sheared to an average target size of about 200 base pairs using a Covaris S2 Focused-ultrasonicator™ in that the entire volume of each exosomal DNA elution (50 μl) was transferred into a 50 μl micro-tube and sonicated for 200 s with an intensity of 2, at duty factor 10% with 200 cycles per burst. Size distribution after sonication was analyzed using the High Sensitivity DNA LabChip Kit (Agilent Technologies) on the 2100 Bioanalyzer (Agilent Technologies).

DNA libraries were prepared from the sheared exosomal DNA preparations as well as the cell-free DNA using the QIAseq cfDNA all in one kit (QIAGEN) according to the manufacturer's recommendations. Quality and size distribution of the DNA libraries was determined using the 2100 Bioanalyzer (Agilent Technologies). Libraries showing a clear peak at approximately 300 bp (corresponding to the length of the DNA fragments plus "technical" nucleotide stretches incorporated into the DNA during the limited cycle PCR) were selected.

Libraries were quantified using the fluorescent dye-based Qubit® dsDNA HS Assay Kit (Invitrogen). In brief, 1 μl of each sample was used to determine the dsDNA concentration (in ng/μl) in comparison to a given standard provided with the kit. The DNA concentration was determined by creating a linear trend line and applying the mathematical equation of linear regression.

Individual libraries were combined in equimolar amounts prior to sequencing to obtain optimum numbers of reads per sample. This way, three different library pools were generated. The library pools were quantified using the fluorescent dye-based Qubit® dsDNA HS Assay Kit (Invitrogen) and concentrations (in nM) were calculated according to the following equation:

$$\text{Conc. [nM]} = (\text{conc. [ng/}\mu\text{l]} * 106) / (660 \text{ g/mol} * \text{average library size [bp]}).$$

Subsequently the libraries were sequenced on an Illumina NextSeq 500 device using the 75 bp paired end chemistry. Sequencing was performed at final concentration of 1.3 pM with a 2% PhiX v3 control library spike-in (Illumina).

Cluster generation sequencing of 2x 75 bp in the paired end mode was performed with each library pool. Prior to sequencing, clusters were generated by bridge amplification. Clusters represent areas on the sequencing flow cell with clonally amplified DNA library fragments. The cluster generation of loaded fragments was carried out by a two-dimensional amplification of bound fragments. Fragments were immobilized on the flow cell, involving two adapters attached at opposing ends of the fragments and complementary slide-anchored primers. By several cycles of flipping and binding of the immobilized fragments to the surrounding primerlawn, followed by amplification, copies of the original fragments were produced and localized in a tight cluster.

After cluster generation, sequencing primers were hybridized to the adapter sequences at the end of the fragments and sequencing was carried out. The sequencing was based on the sequencing by synthesis (SBS) approach and two channel signal detection. During each sequencing cycle, the flow cell was flooded with all four nucleotides (A, C, G, T) which were provided as fluorescently labeled dNTPs, wherein each species of dNTPs was labeled with a different fluorophore than the other three dNTP species.

One base per cycle was attached to the growing antisense strand during strand elongation. Washing away of unbound nucleotides and signal collection from bound fluorescent nucleotides from the different clusters after each cycle in red and green signal channel allowed identification of incorporated nucleotides. Sequencing was operated under the control of the NextSeq® Control Software (NCS).

Primary image processing was performed on the NextSeq® 500 instrument using Real Time Analysis 2.4.11 Software (RTA). Primary data analysis was performed using the bcl2fastq 2.15.04 software package.

The Illumina Sequence Analysis Viewer (SAV) 2.1.8 was used form imaging and evaluation of the sequencing run performance.

Image and signal processing, such as template generation, imaging and base calling, was carried out by the Illumina NextSeq® 500 inherited NCS and RTA software packages applying the FastQ only processing pipeline.

During sequencing every base was assigned with a quality score according to their likelihood of correctness. Raw clusters were determined as well. They were checked for quality issues and low quality clusters comprising a mixed fragment content were filtered out during the processing. The resulting passed filter clusters showed the number of reads finally available for demultiplexing and fastQ generation.

Primary data analysis was performed using the bcl2fastq software tool. This included signal processing from *.bcl to *.fastq files and de-multiplexing of all passed filter reads according to the corresponding detected index sequences. After de-multiplexing, all read 1 files and all read 2 files were concatenated resulting in a total of two *.fastq files per sample, containing quality values and sequence information.

After completion of sequencing runs, technical quality parameters were evaluated using the SAV software. The main quality parameter for a successful sequencing run is the percentage of Q30 bases. Therefore, each base was assigned with a quality Phred score (Q) which is defined as a property logarithmically related to the base calling error probability P according to formula 1:

$$Q = -10 \log_{10} P$$

Quality control of de-multiplexed *.fastq files was performed with the software FastQC version 0.11.5 and summarized in a *.html file. A filtering of sequencing reads with respect to quality was performed.

Prior to analysis of the sequencing data for reads specific for *Borrelia*, all reads were mapped against the host genome and sampled as mapped reads and un-mapped reads, respectively. For mapping, alls *.fastq files were imported into CLC Genomics Workbench version 9.5.3 and mapped against the human genome GRCh38.p7. Mapping was executed with the following settings:

References = GRCh38.p7
Masking mode = no masking
Match score = 1
Mismatch cost = 2
Cost of insertions and deletions = linear gap cost
Insertion cost = 3
Deletion cost = 3
Length fraction = 0.7
Similarity fraction = 0.8
Global alignment = No
Auto-detect paired distances = Yes
Non-specific match handling = map randomly
Output mode = Create stand-alone read mapping
Create report = Yes
Collect un-mapped reads = Yes

Unmapped reads with remaining paired end information and unmapped reads that lost paired end information were exported in separate fastq files.

Prior to the detection of *Borrelia* specific sequences, a quality trimming of host genome unmapped reads was performed using CLC Genomics Workbench. The following settings were applied:

Trim adapter 1sit = Illumina Sequencing Primers
Ambiguous trim = Yes
Ambiguous limit = 2

Quality trim = Yes
Quality limit = 0.05
Use colorspace = No
Create report = Yes
Also search on reversed sequences = Yes
Save discarded sequence = Yes
Remove 5' terminal nucleotides = No
Minimum number of nucleotides in reads = 30
Discard short reads = Yes
Remove 3' terminal nucleotides = No
Discard long reads = No
Save broken pairs = No

Trim Adaptor List: Illumina Sequencing Primers

To test the samples for the prevalence of *Borrelia* derived sequences, all quality filtered unmapped reads were mapped against a *Borrelia* reference collection. The *Borrelia* reference collection was compiled from reference genomes downloaded from NCBI as specified in Table 1.

TABLE 1: *Borrelia sp.* reference genomes

| ID | NCBI *Borrelia* reference genomes | NCBI Accession No. |
|---|---|---|
| 1 | GCF_000008685.2_ASM868v2_*Borrelia_burgdorferi*.gbff.gz | ASM868v2 |
| 2 | GCF_000012085.2_ASM1208v2_*Borrelia_turicatae*.gbffgz | ASM1208v2 |
| 3 | GCF_000019685.1_ASM1968v1_*Borrelia_duttoni*.gbff.gz | ASM1968v1 |
| 4 | GCF_000019705.1_ASM1970v1_*Borrelia_recurrentis*.gbff.gz | ASM1970v1 |
| 5 | GCF_000181895.2_ASM18189v2_*Borrelia_spielmanii*.gbff.gz | ASM18189v2 |
| 6 | GCF_00019215.1_ASM19621v1_*Borrelia_bavariensis*.gbff.gz | ASM19621v1 |
| 7 | GCF_000304735.1_ASM30473v1_*Borrelia_afzelii*.gbff.gz | ASM30473v1 |
| 8 | GCF_000445425.4_SM44542v4_*Borrelia_miyamotoi*.gbff.gz | ASM44542v4 |
| 9 | GCF_000512145.1_ASM51214v2_*Borrelia_pakeri*.gbff.gz | ASM51214v2 |
| 10 | GCF_000568755.1_ASM56875v1_*Borrelia_coriaceae*.gbff.gz | ASM56875v1 |
| 11 | GCF_000739475.1_ASM73947v1_*Borrelia_valaisiana*.gbff.gz | ASM73947v1 |
| 12 | GCF_001660005.1_ASM166000v1_*Borrelia_hermsii*.gbff.gz | ASM166000v1 |
| 13 | GCF_*Borrelia_garnii*.gbff.gz | ASM192254v1 |

The CLC Genomics Workbench was used to map all unmapped reads (single and paired) against the 13 *Borrelia* reference genomes identified in Table 1. Mapping was executed with the following stringent settings:

References = 13 Borrelia genomes
Masking mode = No masking
Match score = 1
Mismatch cost = 2
Cost of insertions and deletions = linear gap cost
Insertion cost = 3
Deletion cost = 3
Length fraction = 0.9
Similarity fraction = 0.8
Global alignment = No
Auto-detect paired distances = Yes
Non-specific match handling = Map randomly
Output mode = Create stand-alone read mappings
Create report = Yes

Collect unmapped reads = Yes

Five different contigs were assembled using the nucleotide sequences that mapped against one of the *Borrelia* genomes. The resulting nucleotide sequences are represented by SEQ ID NO. 1 to SEQ ID NO: 5.

## Claims

**1.** A method for the detection, diagnosis, monitoring or prognostication of a *Borrelia* infection or of the progression of a *Borrelia* infection in an individual, the method comprising the steps of

- recovering extracellular DNA of a bodily fluid sample of the individual to obtain a DNA sample; and
- determining the presence of at least one *Borrelia*-specific DNA biomarker in the DNA sample.

**2.** The method according to claim 1, wherein the bodily fluid sample is selected from the group consisting of a urine sample, a blood sample, a blood plasma sample, a saliva sample, a tears sample, a semen sample and a spinal fluid sample.

**3.** The method according to claim 1 or 2, wherein the extracellular DNA is recovered from exosomes present in the bodily fluid sample.

**4.** The method according to any one of claims 1 to 3, wherein the *Borrelia*-specific DNA biomarker is a DNA molecule comprising a nucleotide sequence consisting of at least 10 consecutive nucleotides of one of the nucleotides sequences set forth in one of nucleotide sequences selected from the group consisting of SEQ ID NO: 1 to SEQ ID NO: 5.

**5.** The method according to any one of claims 1 to 4, the method comprising at least the steps of

a) determining the presence and/or abundance of the *Borrelia*-specific DNA biomarker in the bodily fluid sample, and
b) deciding on the presence or abundance of *Borrelia* in the bodily fluid sample and/or the individual based on the results obtained in a).

**6.** The method according to any one of claims 1 to 5, wherein the presence and/or abundance of the *Borrelia*-specific DNA biomarker is determined in that the DNA of the DNA sample is contacted with at least one nucleic-acid affinity ligand which is specific for the *Borrelia*-specific DNA biomarker.

**7.** A composition for use in the diagnosis, detection, monitoring or prognostication of a *Borrelia* infection or of the progression of a *Borrelia* infection, wherein said composition contains a *Borrelia*-specific nucleic acid affinity ligand.

**8.** The composition according to claim 7, wherein the *Borrelia*-specific nucleic acid affinity ligand is selected from the group consisting at least one oligonucleotide selected from the group consisting of oligonucleotides which

a) comprise a nucleotide sequence as represented in any one of SEQ ID NO: 1 to SEQ ID NO: 5;
b) comprise a nucleotide sequence having a sequence similarity of at least 80% to any one of the nucleotide sequences according to a);
c) comprise a nucleotide sequence being complementary to any one of the nucleotide sequences according to a) or b); and
d) which hybridize to any one the nucleic acid molecules comprising a nucleotide sequence of at least 15 consecutive nucleotides as present in the nucleotide sequences set forth in any one of SEQ ID NO: 1 to SEQ ID NO: 5 under stringent conditions.

**9.** The composition according to claim 7 or 8, wherein the DNA affinity ligand is labelled.

**10.** The composition according to any one of claims 7 to 9, wherein the affinity ligand is a set of oligonucleotides specific for a nucleic acid molecule comprising a stretch of at least 10 consecutive nucleotides present in any one of SEQ ID NOs. 1 to 5.

**11.** Use of a DNA molecule comprising a stretch of at least 10 consecutive nucleotides being present in the nucleotide sequences set forth in SEQ ID NO. 1 to 5 as a *Borrelia*-specific biomarker for diagnosing, detecting, monitoring or prognosticating a *Borrelia* infection.

**12.** A method for diagnosing, detecting, monitoring or prognosticating a *Borrelia* infection comprising at least the step of determining the presence and/or level of a *Borrelia*-specific DNA biomarker in a DNA sample containing extracellular DNA of a bodily fluid sample of a subject.

**13.** The method according to claim 12, wherein the *Borrelia*-specific DNA biomarker is selected from the group consisting of one or more of the genes encoding CheR, PkfB, UvrB, BB0173 and/or BB0037, or fragments thereof.

**14.** The method according to claim 12 or 13, wherein said method comprises the additional step of comparing the measured DNA level to a control level.

**15.** A method of identifying an individual for eligibility for a *Borrelia* infection therapy comprising:

(a) testing in a bodily fluid sample obtained from an individual for the presence and/or abundance of a *Borrelia*-specific DNA biomarker among the extracellular DNA present in said bodily fluid sample;
(b) classifying the levels of abundance of step (a); and

(c) identifying the individual as eligible to receive a *Borrelia* infection therapy where the individual's sample is classified as containing a *Borrelia*-specific DNA biomarker.

Europäisches Patentamt
European Patent Office
Office européen des brevets

# EUROPEAN SEARCH REPORT

Application Number
EP 18 20 2627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2016/118766 A2 (T2 BIOSYSTEMS INC [US]; TOWNSEND JESSICA ANN [US]; SNYDER JESSICA LEE) 28 July 2016 (2016-07-28)<br>* claims 1-3, and 90-92, p. 14, l. 21-25,Fig. 3 and p. 22, l. 24-26 * | 2,3,5-7, 13,14<br>1,4,12, 15 | INV. C12Q1/689 |
| X | WO 2017/139715 A1 (THE TRANSLATIONAL GENOMICS RES INST [US]; ARIZONA BOARD OF REGENTS ON) 17 August 2017 (2017-08-17)<br>* claims 1-14 * | 2,3,5,6, 13,14 | |
| X<br>Y | WO 98/58943 A1 (HUMAN GENOME SCIENCES INC [US]; MEDIMMUNE INC [US]; FRASER CLAIRE [US]) 30 December 1998 (1998-12-30)<br>* claims 1, 19 and p. 35 l. 16-39 * | 8-11<br>4 | |
| Y | WO 2015/112382 A1 (MOREHOUSE SCHOOL OF MEDICINE [US]) 30 July 2015 (2015-07-30)<br>* paragraphs [0049], [0054]; claims 1-10 * | 1,12,15 | |
| | | | TECHNICAL FIELDS SEARCHED (IPC) |
| Y | WO 2018/187521 A2 (UNIV CORNELL [US]) 11 October 2018 (2018-10-11)<br>* claims 1-5, and Fig. 2 [0027] * | 1,12,15 | C12Q |
| Y | VERNON SUZANNE D ET AL: "Analysis of 16S rRNA gene sequences and circulating cell-free DNA from plasma of chronic fatigue syndrome and non-fatigued subjects",<br>BMC MICROBIOLOGY, BIOMED CENTRAL LTD, GB, vol. 2, no. 1, 23 December 2002 (2002-12-23), page 39, XP021014820,<br>ISSN: 1471-2180, DOI: 10.1186/1471-2180-2-39<br>* abstract * | 1,12,15 | |

The present search report has been drawn up for all claims

2

| Place of search | Date of completion of the search | Examiner |
|---|---|---|
| Munich | 10 December 2018 | Lapopin, Laurence |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 20 2627

This annex lists the patent family members relating to the patent documents cited in the above-mentioned European search report. The members are as contained in the European Patent Office EDP file on
The European Patent Office is in no way liable for these particulars which are merely given for the purpose of information.

10-12-2018

| Patent document cited in search report | Publication date | Patent family member(s) | Publication date |
|---|---|---|---|
| WO 2016118766 A2 | 28-07-2016 | EP 3248011 A2 | 29-11-2017 |
| | | US 2018171388 A1 | 21-06-2018 |
| | | WO 2016118766 A2 | 28-07-2016 |
| WO 2017139715 A1 | 17-08-2017 | NONE | |
| WO 9858943 A1 | 30-12-1998 | AU 8151898 A | 04-01-1999 |
| | | AU 8153498 A | 04-01-1999 |
| | | CA 2294568 A1 | 30-12-1998 |
| | | CA 2304925 A1 | 30-12-1998 |
| | | EP 1009859 A1 | 21-06-2000 |
| | | EP 1012157 A1 | 28-06-2000 |
| | | WO 9858943 A1 | 30-12-1998 |
| | | WO 9859071 A1 | 30-12-1998 |
| WO 2015112382 A1 | 30-07-2015 | CN 106029898 A | 12-10-2016 |
| | | EP 3097202 A1 | 30-11-2016 |
| | | WO 2015112382 A1 | 30-07-2015 |
| WO 2018187521 A2 | 11-10-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **STRYER.** *Science,* 1968, vol. 162, 526-533

SEQUENCE LISTING

<110> TBD-diagnostics GmbH

<120> Diagnosis of Lyme disease

<130> 62 638 V

<160> 5

<170> PatentIn version 3.5

<210> 1
<211> 852
<212> DNA
<213> Borrelia burgdorferi

<400> 1
atgaacacga atcaaaacaa attcaacctt aatataaata taactaagga cgaactttca 60

agattaataa aaatagttta caataatttt ggaatcaatc tcagcgaaaa aaaaaagctg 120

ctaattgaaa gcagattatc atctcttctg aaagttaaag gttttaaaaa ttttactgaa 180

tacatcaact ttttagaaaa aagcactgga aatattcaat taatcgaatt aatagacaaa 240

atatcaacaa atcataccta ttttttcaga gaatctaagc attttgattt tttaaataat 300

aaaatattac ccaaacttac tgaaaaaata ttaaactcag aaaactcaga aattagaata 360

tggtcagctg gctgttcaag tggtgaagag ccttatacaa ttgcaatgat gctaaaagaa 420

tatatggaac ataatagagt aaattttaaa gtcaaaatct tagcaacaga catttcaatt 480

agcgttctaa atgaggctcg tgaagggatt tatcctgaag atcgcataat aaatttgccc 540

aaatatttaa aaattaaata tttaaatcaa cttcaagatg ataaatttca agttaaagaa 600

attttaaaaa aaatggttta ctttaaaaaa ttaaatttaa tggatgagaa atttccattt 660

agcaaaaaat ttgacctaat attttgcaga aatgtcatga tctattttga tgaaaaaact 720

aggaataacc ttgccaataa attcaactcc taccttaaaa aggattctta tcttttaatt 780

ggacattcag aaacaatcag aggaaacaaa aatcttgaat acataatgcc tgccacctat 840

aaaaagaatt aa 852

<210> 2
<211> 924
<212> DNA
<213> Borrelia burgdorferi

<400> 2
ttgatatata ctctaacact caatccttct gtggattata aaatagtttt aaaagaattt 60

caggaagaaa gtcttaatta tgctttaaat aacaattttt ttgctggcgg taagggaata 120

aatgtaagca ccgttcttaa aaatttagga aaacctagta cggctttggg atttttggga 180

ggttttacgg gtgattatat aagattttct cttgattcta ggggcataaa aaacgatttt 240

attaaaataa aatatgatac aagattaaat attaaaatga tagcaaatgg cagagaaaca 300

gaaattaatg ccaattctcc agatatttct gagaatgaat ttgaactttt gaaaaataaa 360

cttaaaaatt tagcaaataa tagtacatta gtgatgtcag gaagtgttcc cgcagctctt 420

ggtgaggatg catacaatga aatagctaat agcatttcta atgatgttaa gcttatcatt 480

gataccagtg gcaaaccttt gcgaaaaatt cttagattaa atccctttt aataaagcct 540

aatatttatg aacttgaaga tctttttaat gctaaatttg attctacaaa agaattgatt 600

aaaatcggaa aaaatcttgt agaaagtggg gttcaaaaca ttataatttc catgggaagt 660

gacggagcta tttttattgg cggcaaaaat gttgctttta gggcctttgt tcctaagatt 720

aattttgtta gcaccattgg agcaggagac tctgtgattg ccgggtttgt atatgctttt 780

gataatggaa gtactttaga ggattcattt aaatttggtg ttgcagctgg tacagctacg 840

gcattaaaag gcaatctttg tgaatttcaa gatgttaaaa agatgctttg tcagattagg 900

gttgaggata tttacacttc ttaa 924

<210> 3
<211> 1995
<212> DNA
<213> Borrelia afzelii

<400> 3
atgatagatt ttttttgaa atcagaatat cttcccgctg gtgatcagcc taaggcaata 60

aaagagattg aaaattctat tttgctaggg aataagtatc aaacattaaa aggtgttacg 120

gggagtggaa agacttttac aattgcaaat ataattaaaa acttaaacag acctgcctta 180

gttgtaagtc ataacaaaac attagcagcg caactttata gagagtttaa agactttttt 240

ccaaacaatg ctgttgaata ttttgtttct tattatgatt attatcagcc agaatcctat 300

gtgccttcaa aggatttatt tattgaaaaa gaagctacta ttaattctga gatagaaatt 360

aagcgaataa gaacggtaac gtctcttgct aaaagacgag atgttattgt tgttgcaact 420

gtatcttcaa tttatgctct tggatctcca gatttttca aaaaatcagc gcgagaattt 480

tttgtaggtc aaagaatttc tattaaagaa atatcagata tttttgtaga gctttattat 540

gagagaactt tgataaatct agaaagagat aaattttcaa ttaagggaga tattattgaa 600

atttggccaa gcagtgagca tggagagttt gcgtatcgaa tttgtttgga ttttgatgag 660

attgttaaaa tatataggat tagttcattt tctaaaaaaa atttaggagc tacgaatagt 720

tttactcttt ttgctaaatc ttattttgta attccttatc aaaacgtatt agaagcgata 780

cctaaaatat cttatgattt agatctccaa tgtcaatatt ttaaagataa tggcaagctt 840

gtagaagccg agagacttaa gcagagggtg gagtatgatt tggaaatgct tagagaaaca 900

ggattttgct ctggcattga aaattattct aaatatttga gtggaagcac aatgggaaga 960

ccttattgtc tttttgattt tttcccaaaa gattacttat tgtttgtaga tgaatctcat 1020

gttacattgc cacaatttag ggggatgtat aatggagatt attctagaaa attgaatctt 1080

gttaacttcg gatttagact tcctgcagcg cttgaaaata gaccccttaa gtatgatgaa 1140

tttgacgcat taattaatca ggttgtgttt gtatctgcaa ctccaggttt tgaagagaat 1200

gaaaagagta gtgtgactgt tgatcaaata attcgtccca caggccttgt tgatcctgaa 1260

attattacta ggcattctga tgggcaaatg gaagatcttt atatcgagat tcaaaaaaga 1320

gtagctctta aagagcgggt tttaattact actttgacaa aaaaaatgtc tgaggatttg 1380

actgaatatt tagtaactct tggtgtaaag gcaaaatatt tacattcaga gcttgacact 1440

cttgaaagag tagaagttat ttcattgctt agaaaatctg aaattgatgt tattgttggt 1500

attaacttgc ttagggaagg cttagatatt ccagaagtat ctcttgttgc aatattagat 1560

gccgataaag tagggttttt aagatctgct acttcattaa tacaaacaat tggcagggct 1620

gctagaaatt ctaatggact tgtaataatg tattacgaca aaataagtgt agctatgcgg 1680

gaggcaattg aggagactaa tagaagacgt caaattcaga ttgattataa taaaaaaaat 1740

aatattactc ctaagacaat tgttaagaag attcaaaata ttttagaaaa agaactaaat 1800

aacaaaaata aaaatattag ctatgacttt gaaaaaatgg tttcaggtga gaaattgtct 1860

aaaaaaaagc ttattgataa gcttaaattt gaactagaag aagctgttaa tgatgaaaga 1920

tttgaagatg caattgtttt aagagataaa ataaaagagc ttggtagcaa aattagtgtg 1980

gctcgtaata aatga 1995

<210> 4
<211> 1002
<212> DNA
<213> Borrelia burgdorferi

<400> 4
tcatagtatc tcttttaaga aaatttttga aaaaataaaa taaacaagca ataagcaaaa 60

cgctaaaact aaaaattctt tataaatatc tttattgtcc acagctattt taatttttct 120

ctccaaattt tccttttttg aaaaatcttg aattgcaaat tgaaaagaaa aatcatcatt 180

aaccgaataa aaaagccccc ccgttttatt tgaaatctcc acaagcatac taggatcata 240

aacctcttta aaacttccct gataaaattt tccagatctt aatttaaact caacactaaa 300

ttcttcagaa cttccaatac caatagaata aatcttaaca tttaaacctt gagcaagatt 360

gatcacttga tctttatraa tctcatctga attaacaacc ccatctgtta aaaccactat 420

tgatctttta agagcctcag aatgctttaa atgagacagc gcaatagaaa tacctagccc 480

taaagcagaa ccattgccaa gatccataat ataaatatca tctagctttt tattaaaaaa 540

ttccctatct gttgttatag gcactactat tgaagcatct tttgcaaaag ctaccaaacc 600

aatattatca ttttcacgtt gagaaataaa acctctaatc aattcttttg aaaattcaag 660

tctatttttg gaagaaaact caacagcccc catactaggc gatatgtcaa gaacaatgac 720

aatgtcagca ccagcactaa gatgtatcat cttctttttt gaaactgaag gacctgctaa 780

agcaaacacc ataaccattg cagctaaata taaaaaagag taagtaaaaa aatacatcaa 840

atttaatcta taatccttaa gttttaaaga gttgaaattg ccgtaaagcg atattggaaa 900

ctttatcttg cctcctctat tttttaaaaa atgattaaag taaattatta aaggaaaaat 960

tactaataaa aacaaatata aaggctcatt aaatgttaac at 1002

<210> 5
<211> 753
<212> DNA
<213> Borrelia burgdorferi

<400> 5
ttaaatttta atagtttcaa gctttttaac tatttgatct ctaataatac tagtgagatt 60

ctctttttca tactcactta attttaaaac atctatcaaa ggatggacat gaatataaac 120

agacagtcca gaattaaaaa taatattttt aataaaaaag ttattagtat tataaagagt 180

aacaggaaga attgatgttc ctgtttttaa tgccatctta attgaacctt ttttaaaaac 240

tctagtatcc ccccctctat ttctagttcc ttcggggaaa attccaatag atctaccttc 300

tctcataacc tttattgcct taacctcagc agcagcagca gatcttatgc tccttctatt 360

aacaaaaata acacccataa caatcaaaac aatattcaca aaaggaatcc taagcaacga 420

atgttttgcc aatattacaa atgggcaggc aaaagtataa ataaaaatta aaggatccat 480

agccgcaata tgatttccca ttattattac attgcttttt ttaggaatat tttcagaacc 540

cgtaacaata attttaattc cagcaagcca taagctaatt ttaatgcaag ctcgcatcat 600

aatgaaacta agcctaacaa cataacgttc aattaaaaaa attttacaaa ccaggtaaaa 660

aggaaataaa aaaaaaatta aaatcaaaaa aaacaataaa acattaaaat aagttataat 720

acttctcaat attttcataa aaactattat cat 753